# EUROPEAN PATENT SPECIFICATION

(11) **EP 2 740 481 B1**
(45) Date of publication and mention of the grant of the patent: **11.07.2018**
(21) Application number: 12793897.5
(22) Date of filing: 31.01.2012
(51) Int. Cl.: A61K 35/64, A61P 19/10, A61K 33/06

(54) **METHOD FOR FILLING BONE CAVITY FORMATIONS WITH CALCIUM**
VERFAHREN ZUM BEFÜLLEN VON KNOCHENINNENRÄUMEN MIT CALCIUM
PROCÉDÉ DE REMPLISSAGE DE FORMATIONS CREUSES DES OS AVEC DU CALCIUM

(30) Priority: 31.05.2011 RU 2011121932
(43) Date of publication of application: 11.06.2014
(73) Proprietor: Obshchestvo S Ogranichennoj Otvetstvennost'ju "PARAFARM", Penza 440026 (RU)
(72) Inventor: STRUKOV, Villorij Ivanovich, Penzenskaya obl. 440011 (RU); TRIFONOV, Vjacheslav Nikolaevich, Penzenskaya obl. 442960 (RU); ELISTRATOVA, Julija Anatol'evna, Moscow 127549 (RU); ELISTRATOV, Konstantin Gennad'evich, Penzenskaya obl. 440061 (RU); KURUS' Natal'ja Vjacheslavovna, Penzenskaya obl. 440026 (RU)
(74) Representative: Engel, Christoph Klaus
(86) International application number: PCT/RU2012/000049
(87) International publication number: WO 2012/166003

(56) References cited:
- KR-A- 20050 040 660
- RU-C1- 2 233 666
- RU-C1- 2 338 551
- RU-C1- 2 412 616
- LAZARYAN D S ET AL: "Drone breed-base tablet apilar eliciting anabolic and actoprotective effect", WPI / THOMSON,, vol. 2004, no. 59, 10 August 2004 (2004-08-10), XP002687607,
- E. BRAUNVALDA ET AL.: 'Vnutrennie bolezni. V 10 knigakh. Kniga 9: Per. s angl. Red. Pervogo izdaniya T. R. Kharrison/Pod red.' MEDITSINA 1997, page 414,416, XP008172773

## Description

The invention relates to medicine, in particular to methods for combating the effects of calcium deficiency in the skeletal system of the body, which manifests in the form of an increase in the quantity and size of cavities in metaphyseal (trabecular) bone sections and for preventing such manifestations of calcium deficiency.

The existing methods for preventing and treating calcium deficiency in the body aim to saturate bone tissue with calcium. To that end, various calcium-containing preparations as well as vitamin D and hormonal preparations (such as testosterone) are used.

These methods have a number of significant disadvantages. Although calcium is not a toxic substance and no maximum allowable doses for administration have been established for it, prolonged use of significant quantities of calcium may increase the risk of stroke. When calcium preparations are being used, calcium can be supplied to other organs and systems as well as to bones, and in particular, can calcify calculi in the kidneys, which in turn leads to kidney function issues.

Similarly, there is a risk of exceeding the safe quantity of vitamin D in the body. In several cases the use of hormonal preparations is undesirable or completely excluded for medical reasons. Artificially increasing testosterone in females (even for apparently healthy females) is generally undesirable. It is also important to consider the fact that using calcium-containing preparations either separately or in combination with vitamins and/or hormones, in addition to all of the above-mentioned side effects and contra-indications, only makes it possible to increase mineralisation of bone tissue. Existent and newly-formed cavities in metaphyseal (trabecular) bone sections remain unchanged or the quantity and sizes thereof may continue to increase. These cavities reduce bone strength and increase the risk of injury at various loads.

While the applicant regularly takes part in research in this field, they are unaware of any methods that would provide for the desired result.

The current methods for increasing the calcium content in bone tissue in the body makes it possible to increase bone mineralisation but not to fill cavities therein.

The proposed solution aims to reduce and eliminate, where possible, existing cavities and to keep new cavities from forming. The desired result is achieved by providing for the supply of drone brood in combination with calcium to a body.

As experiments have shown, regular drone brood treatment in combination with supplying calcium to a body provides for the desired results.

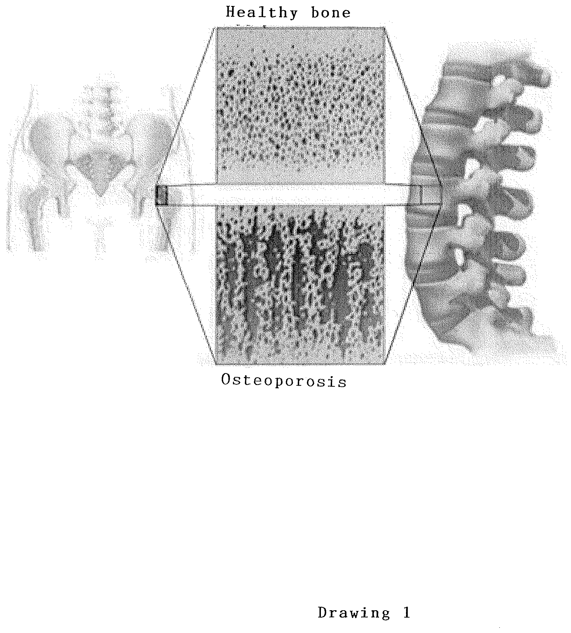

So as to have a better visual understanding of the area for using this solution, an illustration is provided in drawing 1, which shows the location of cavities in the bone; showing both healthy bone and bone in a body that has been diagnosed with osteoporosis.

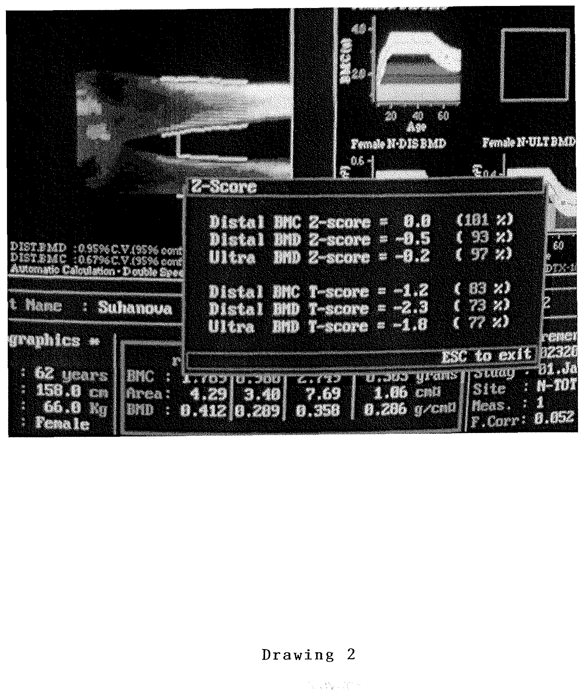

Drawing 2 shows the result of an X-ray examination prior to the commencement of treatment.

Two black cavities can be seen at the top and at the bottom of the bone.

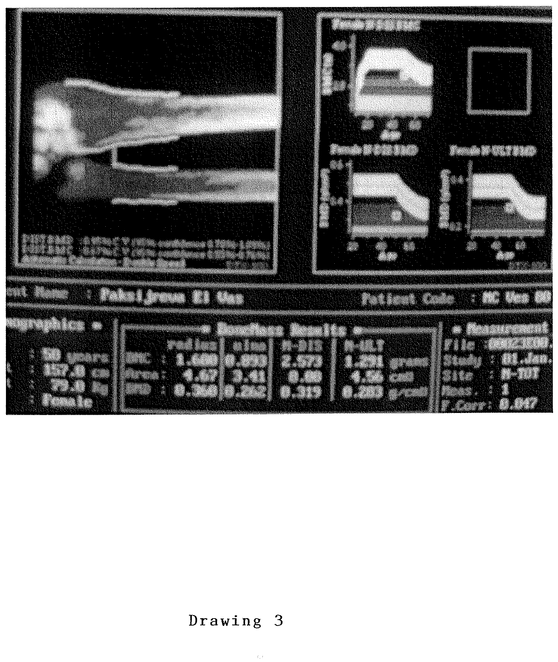

Drawing 3 shows the results of an X-ray examination after a course of treatment. The cavities have been filled and have become blue.

The research was carried out using an X-ray absorption method on NORLAND, OSTEOMETR DTX-100 and 200 apparatuses on a group of 10,000 patients.

The calcium in the cavities is predominantly in the form of calcium hydroxyapatite.

In addition to providing for the supply of calcium to the body in common food products, various calcium-containing preparations can be used including calcium carbonate, calcium citrate, calcium gluconate, calcium aspartate, calcium ascorbate, calcium amino chelate, calcium fumarate, calcium succinate, calcium phosphate, and calcium citrate, any one of these or any combination thereof.

During the experimental confirmation of the results, drone brood was given to patients orally in powder form, tablet form or capsule form either as the individual active agent or in combination with calcium-containing compounds.

The desired result can be achieved more quickly or more slowly depending on the individual properties of a particular body, the amount of calcium required in food and the quantity and size of the cavities, but the result is achieved in any case. It has been seen that 100% of patients achieved practically significant results over an extended course of treatment (nine months).

## Claims

1. Combination of drone brood and calcium for use in the treatment of cavities in metaphyseal (trabecular) bone sections by filling calcium and prevention of its excretion from these bones.

2. Combination for the use of claim 1, wherein drone brood is in the form of powder, tablets or capsules.

3. Combination for the use of claim 1 or 2, wherein calcium is presented as a calcium-containing preparation.

4. Combination for the use of claim 3, wherein the calcium-containing preparation is a calcium compound or a combination thereof chosen of the following group of:
- calcium carbonate,
- calcium citrate,
- calcium gluconate,
- calcium aspartate,
- calcium ascorbate,
- calcium aminochelate,
- calcium fumarate,
- calcium succinate,
- calcium phosphate,
- calcium citrate.

## Patentansprüche

1. Verbindung aus Drohnenbrut und Calcium zur Verwendung bei der Behandlung von Hohlräumen in metaphysären (trabekulären) Knochenabschnitten durch Einfüllen von Calcium und Verhinderung seiner Absonderung aus diesen Knochen.

2. Verbindung zur Verwendung gemäß Anspruch 1, wobei Drohnenbrut in Form von Pulver, Tabletten oder Kapseln vorliegt.

3. Verbindung zur Verwendung gemäß Anspruch 1 oder 2, wobei Calcium in Form eines calciumhaltigen Präparats bereitgestellt wird.

4. Verbindung zur Verwendung gemäß Anspruch 3, wobei das calciumhaltige Präparat eine Calciumverbindung ist oder eine Kombination von solchen, gewählt aus der folgenden Gruppe:
- Calciumcarbonat,
- Calciumcitrat,
- Calciumgluconat,
- Calciumspartat,
- Calciumascorbat,
- Calciumaminochelat,
- Calciumfumarat,
- Calciumsuccinat,
- Calciumphosphat,
- Calciumcitrat

## Revendications

1. Combinaison de couvain de mâles et de calcium pour une utilisation dans le traitement de cavités dans des sections osseuses métaphysaires (trabéculaires) par le remplissage de calcium et la prévention de son excrétion de ces os.

2. Combinaison pour l'utilisation selon la revendication 1, dans laquelle le couvain de mâles est sous la forme d'une poudre, de tablettes ou de capsules.

3. Combinaison pour l'utilisation selon la revendication 1 ou la revendication 2, dans laquelle le calcium est présenté comme une préparation contenant du calcium.

4. Combinaison pour l'utilisation selon la revendication 3, dans laquelle la préparation contenant du calcium est un composé de calcium ou une combinaison de ceux-ci choisie parmi le groupe suivant constitué de :
- carbonate de calcium,
- citrate de calcium,
- gluconate de calcium,
- aspartate de calcium,
- ascorbate de calcium,
- aminochelate de calcium,
- fumarate de calcium,
- succinate de calcium,
- phosphate de calcium,
- citrate de calcium.
